# EUROPEAN PATENT APPLICATION

(11) **EP 1 997 820 A1**
(43) Date of publication of application: **03.12.2008**
(21) Application number: 06705735.6
(22) Date of filing: 09.03.2006
(51) Int. Cl.: C07D 471/04, A01N 43/90, A01P 7/00

(54) **PREPARATION METHOD AND USE OF COMPOUNDS HAVING HIGH BIOCIDAL ACTIVITIES**

(71) Applicant: East China University of Science and Technology, Shanghai 200237 (CN)
(72) Inventor: LI, Zhong, 200237 Shanghai (CN); QIAN, Xuhong, 200237 Shanghai (CN); SHAO, Xusheng, 200237 Shanghai (CN); XU, Xiaoyong, 200237 Shanghai (CN); TIAN, Zhongzhen, 200237 Shanghai (CN); HUANG, Qingchun, 200237 Shanghai (CN)
(74) Representative: Modiano, Micaela Nadia
(86) International application number: PCT/CN2006/000360
(87) International publication number: WO 2007/101369

(57) **Abstract**

The present invention discloses a kind of nitromethylene derivatives as well as their preparation method and their uses. The insecticidal activity tests show that the nitromethylene derivatives of the present invention not only show high insecticidal activities against insects with piercing-sucking type or scratching type mouthparts, such as aphid, leafhoppei, plant hopper, thrips and white fly and their resistant strains, but also show high insecticidal activities against Lissorhoptrus oryzophilus, carmine spider mite, and they can also be used to prevent sanitary pest, and white ant.

## Description

### FIELD OF THE INVENTION

The present invention relates to nitromethylene derivatives, the preparation methods thereof and the uses thereof.

### BACKGROUND OF THE INVENTION

Nicotine, which is an alkaloid from the extraction of tobacco leaves, serves as a natural insecticide, and it has a unique mechanism of action, whose target is the postsynaptic nicotinic acetylcholine receptor (nAchRs). However, it has low insecticidal activity to insects while it has high toxicity to humans. In middle 1980s, Bayer AG successfully developed the first new nicotine insecticide---Imidacloprid. After Imidacloprid, a series of nicotine insecticides such as Thiacloprid, Clothianidin, Thiamethoxam, Acetamiprid, Nitenpyram and Dinotefuran were developed. Neonicotine insecticides are the most important class of insecticides due to their high potency, low toxicity to mammalian and aquatic animals, broad insecticidal spectrum, good systemic properties and proper field stability.

Compared to Imidacloprid, nitromethylene compounds have higher binding affinity and insecticidal activity. Whereas its use as an insecticide has been restricted by its instability to light and low LogP value. Li Zhong et al did some structural modifications on nitromethylene compounds which have high activity and successfully discovered a series of compounds having high insecticidal activities by introducing a ring structure to the nitromethylene compound and controlling the space orientation of nitro group. These compounds have been disclosed in the Chinese Application No. 200410084457.1 (Publication No. CN1631887). However, the complex preparation methods and the high cost limit the use of these compounds.

Therefore, there is an urgent demand to develop compounds which have higher insecticidal activities and can be prepared with low cost and relatively simple preparation methods.

### SUMMARY OF THE INVENTION

The object of this invention is to provide a kind of nitromethylene derivatives which have high insecticidal activities, low cost and can be prepared with relatively simple methods, and to provide the preparation methods thereof, and the uses thereof.

According to the first aspect of this invention, it is provided a nitromethylene derivative of formula (I). 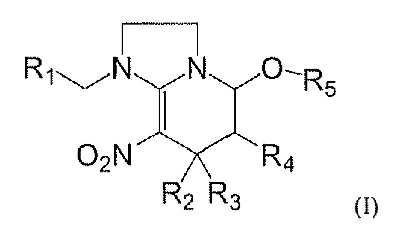
wherein R₁ is a 5 or 6-membered heterocycle containing nitrogen, oxygen and/or sulfur, or halogenated 5 or 6-membered heterocycle containing nitrogen, oxygen and/or sulfur;
R₂ is a hydrogen atom, C₁₋₄ alkyl group, C₁₋₄ alkoxyl group or aryl group; R₃ is a hydrogen atom, C₁₋₄ alkyl group, or C₁₋₄ alkoxyl group; R₄ is a hydrogen atom, C₁₋₄ alkyl group, C₁₋₄ alkoxyl group or aryl group;
R₅ is a hydrogen atom, a saturated or unsaturated C₁₋₈ hydrocarbyl group, saturated or unsaturated C₁₋₈ halogenated hydrocarbyl group, -CH₂CH₂OCH₂CH₃, -CH₂CH₂OCH₃, saturated or unsaturated C₁₋₈ hydrocarbyloxy;

Preferably, R₁ is one of pyridyl, thiazolyl, pyrimidinyl, tetrahydrofuryl, oxazolyl, or the chloride thereof; R₅ is a hydrogen atom, saturated or unsaturated C₁₋₅ alkyl group, saturated or unsaturated C₁₋₅ chlorinated or fluorinated alkyl group, -CH₂CH₂OCH₂CH₃, or -CH₂CH₂OCH₃, saturated or unsaturated C₁₋₈ alkoxyl group.

More preferably, R₁ represents

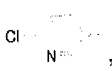

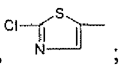

More preferably, R₅ represents a hydrogen atom or saturated or unsaturated C₁₋₅ hydrocarbyl group;

More preferably, R₂ represents a hydrogen atom, C₁₋₃ alkyl, or C₁₋₃ alkoxyl;

More preferably, R₃ represents a hydrogen atom, C₁₋₃ alkyl, or C₁₋₃ alkoxyl;

More preferably, R₄ represents a hydrogen atom, C₁₋₃ alkyl, or C₁₋₃ alkoxyl,

Preferably, the said aryl group is substituted or unsubstituted phenyl group, the said substituent is selected from C₁₋₃ alkyl, C₁₋₃ alkoxyl, halogen, -NH₂, -NO₂ or -OH.

One kind of especially preferable compound has the structure of formula A (i.e. R₃=R₅=H): 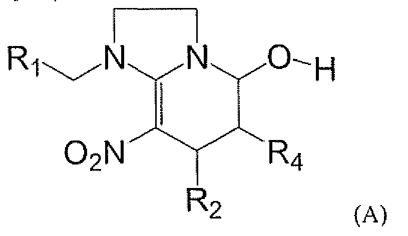 wherein R₂, R₄ are defined as above.

More preferably, R₁ represents

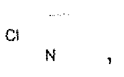

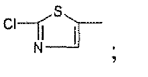

More preferably, R₂ represents methyl;

More preferably, R₄ represents a hydrogen atom, C₁₋₃ alkyl or C₁₋₃ alkoxyl;

According to the second aspect of this invention, it is provided a pesticidal composition comprising 0.0001wt%-99.9wt% of the derivative of formula (I) of the present invention or an agriculturally acceptable salt thereof in mixture with an agriculturally acceptable carrier or diluent.

In one preferred example, the concentration of the said derivative of formula (I) or an agriculturally acceptable salt thereof is 10-500 ppm, more preferably 20-100 ppm.

In another preferred example, the formulation of the said composition can be a variety of conventionally used formulations in pesticides, such as bait formulation and the like

According to the third aspect of this invention, it is provided a method of controlling pests which comprises applying to plant seeds, plant leaves and/or plant fruits or the places where the plant is growing or is expected to be grown an insecticidally effective amount (such as 10-500ppm, more preferably 20-100 ppm) of the derivative of formula (I).

In another preferred example, the pests are selected from insects with piercing-sucking type or scratching type mouthparts, Lissorhoptrus oryzophilus, carmine spider mite, sanitary pest (such as blattella germanica, dermatophagoides pteronyssinus, xenopsylla cheopis and ants) and white ant.

According to the fourth aspect of this invention, it is provided the use of the derivative of formula (I) in preparing chemical insecticide for agriculture.

According to the fifth aspect of this invention, it is provided the preparation method of the derivative of formula (I), the said method includes the following procedures:
(a) In an appropriate solvent, a compound of the formula (II) reacts with a compound of formula (III) at 60-100 °C to form a compound of formula (IV); 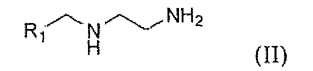 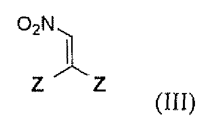 wherein Z represents Cl, -OR', -SR' in which R' represents C₁₋₃ alkyl group (more preferably R'=methyl) 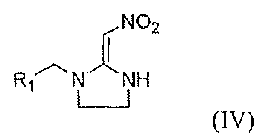
(b) In an appropriate solvent, the compound of formula (IV) reacts with a compound of formula (V) in the presence of an acid catalyst at 0-90 °C to form a compound of formula (VI). 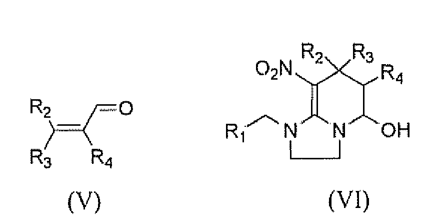
(c) In an appropriate solvent, the compound of the formula (VI) reacts with a compound of formula (VII) in the presence of a catalytic amount of acid at 30-70 °C to form a compound of formula (I).

   R₅OH (VII)
wherein, R₁, R₂, R₃, R₄ and R₅ are defined as in claim 1;

Provided that when Z represents -OR' or -SR', any two or three steps in procedures (a), (b) and (c) can be combined into one step.

### Mode of currying out the invention

After intensive and extensive study, the inventors add a ring structure to the existing nitromethylene compounds in order to enhance its light stability and liposolubility. Furthermore, this invention can control the space orientation and liposolubility by the substituent linked to an ether bond. Additionally, based on screening of many compounds, a type of derivatives of formula (I) with high insecticidal activities, whose preparation method was simple, were picked out and the invention was thus completed.

Concretely speaking, the present inventors provide three preparation methods for the derivative of formula (I), which greatly simplify the synthetic routes and reduce the cost for preparing the compounds by reducing the reaction temperature and changing the solvent, thus improving the actual value of the compounds.

Furthermore, the bioassays show that the compound of formula (I) is an insecticide with wide insecticidal spectrum. The nitromethylene derivatives of the present invention not only show high insecticidal activities against insects with piercing-sucking type or scratching type mouthparts, such as aphid, leafhopper, plant hopper, thrips and white fly and their resistant strains, but also show high insecticidal activities against Lissorhoptrus oryzophilus, carmine spider mite, and they can also be used to prevent sanitary pest, white ant and the like.

### Preparation methods

The nitromethylene derivatives of the present invention can be synthesized by the following schemes:

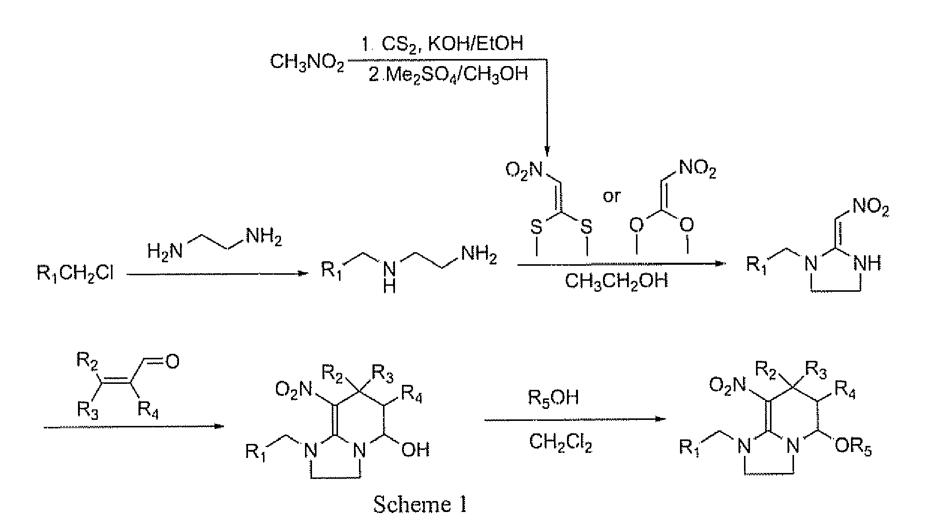

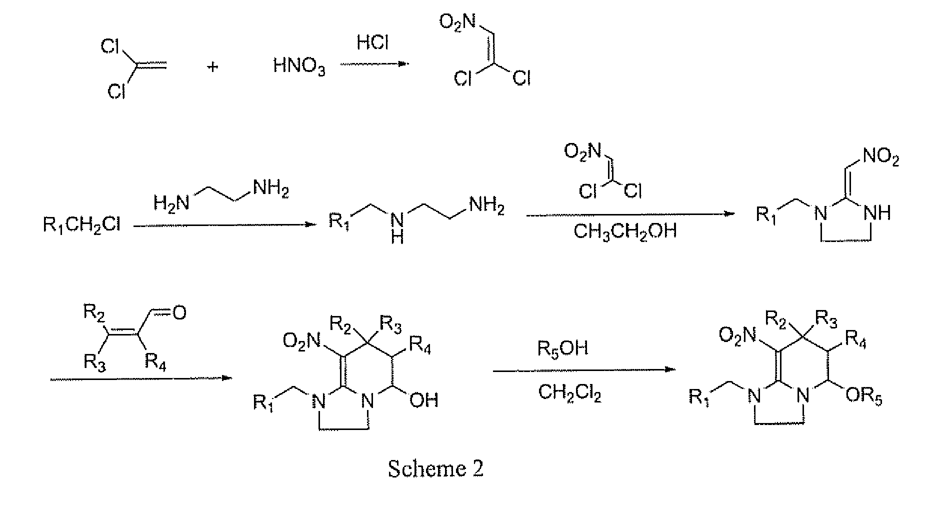

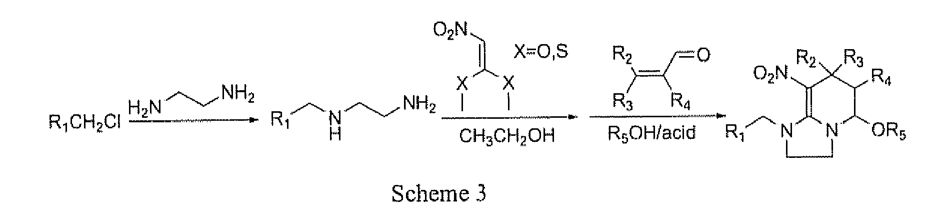

### Scheme 1:

(1) A mixture of nitromethane and carbon bisulfide, which is placed in a three-necked flask and dissolved in an alcohol, is added dropwise to the solution of potassium hydroxide in an alcohol at room temperature. The reaction is carried out at the temperature range of 0-35 °C for 2-10 hours. The solid is filtered out to afford a crude product potassium 2-nitroethene-1,1-bis(thiolate) which is a brown yellow powder.
(2) Potassium 2-nitroethene-1,1-bis(thiolate) is dissolved in an alcohol, and then to the resulting mixture is added dropwise dimethyl sulfate solution. The reaction is stirred at room temperature for 2-8 hours. The precipitated solid is filtrated out to obtain a crude product 1,1-dimethylthio-2-nitroethene, which is a light brown yellow powder.
(3) The solution of 2-chloro-5-(chloromethyl)pyridine in acetonitrile is added dropwise to a solution of diamine whose mole amount is 5-10 times of that of 2-chloro-5-(chloromethyl)pyridine. The reaction is carried out at the temperature range of 0-50°C for 5-10 hours. Water is added to the reaction mixture, which is then extracted with chloroform and the solvent is removed by rotary evaporation. The diamine is removed as far as possible by rotary evaporation, and then N¹-((6-chloropyridin-3-yl)methyl)diamine is obtained.
(4) The mixture of N¹-((6-chloropyridin-3-yl)methyl) diamine and 1,1-dimethylthio-2-nitroethene is dissolved in ethanol and refluxed for 4-8 hours to obtain the product nitromethylene compound.
(5) In the presence of an acid catalyst such as hydrochloric acid, sulfuric acid, acetic acid, trifluoroacetic acid and the like, nitromethylene compound reacts with olefin aldehyde to obtain a new nitromethylene compound having a hydroxyl group.
(6) In the presence of a catalytic amount of hydrochloric acid, the new nitromethylene compound having a hydroxyl group and various alcohols whose amounts are five times of that of the new nitromethylene compound are refluxed in dichloromethane to give an ether compound containing nitromethylene structure. This method can enhance the yield by 30-50% and reduce side reactions.

In the presence of an acid-binding agent such as organic or inorganic alkali, for example pyridine, triethylamine, potassium carbonate, potassium hydroxide and the like, the mixture of the new nitromethylene compound having a hydroxyl group and various acyl chlorides or acid anhydride is refluxed to obtain the target compound.

### Scheme 2:

(1) To a mixture containing 36% hydrochloric acid and 65% nitric acid in a three-necked flask is added dropwise 1,1-dichloroethene, and then the mixture is extracted with dichloromethane. The oil layer is washed with water, and then alkaline is added. After the reaction is complete, the reaction mixture is extracted with dichloromethane, and the oil layer is washed with water, dried and filtrated, evaporated to obtain yellow liquid.
(2) The steps 3, 4, 5, 6 are the same as that in Scheme 1. This method can increase the yield by 20-40%.

### Scheme 3 (one-pot method):

The steps 1, 2, 3 are the same as that in Scheme 1, and the following steps are performed by one-pot method:
N¹-((6-chloropyridin-3-yl)methyl)diamine and 1,1-dimethylthio-2-nitroethene are dissolved in a corresponding alcohol and refluxed for 4-8 hours, and then olefin aldehyde and acid (hydrochloric acid, sulfuric acid, acetic acid, trifluoroacetic acid, and the like) are added, refluxed to obtain the corresponding ether derivatives bearing nitromethylene scaffold. This method can increase the yield by 20-40% since one separation step is eliminated.

The invention is further illustrated by the following examples. It should be appreciated that these examples are only intended to illustrate the invention, but not to limit the scope of the invention. For the experimental methods in the following examples, they are performed under routine conditions, or as instructed by the manufacturers, unless otherwise specified. The percents and parts are based on weight unless otherwise specified.

### EXAMPLE 1

### Synthesis of 1-((6-chloropyridin-3-yl)methyl)-7-methyl-8-nitio-1,2,3,5,6,7-hexa hydroimidazo[1,2-a]pyridin-5-ol (compound 1)

### (1) Synthesis of potassium 2-nitro-ethene-1,1-bis(thiolate)

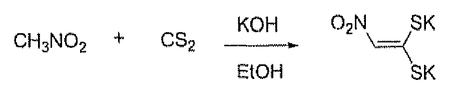

4g (0.03mol) of nitromethane and 6ml (0.05mol) of carbon bisulfide were placed in a 100 ml three-necked flask and 10ml of ethanol was added as a solvent, and then the solution was stirred. To the resulting solution was added slowly and dropwise the solution of 8g (0.14mol) of potassium hydroxide in 40ml of ethanol at room temperature over nearly 30min. Since the reaction was exothermic, the rate of addition depended on the reaction temperature, which was preferably controlled between 30-35°C. After the addition was complete, the mixture was further stirred for 2 hours, filtered to obtain a crude product, which was a brown yellow powder in 72% yield.

### (2): Synthesis of 1,1-dimethylthio-2-nitroethene

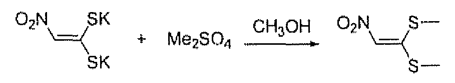

To a solution of 2g (0.0094mol) of potassium 2-nitroethene-1,1-bis(thiolate) in 10ml of dried methanol in a round-bottomed flask, 0.0187mol of dimethyl sulphate was added. The mixture was then stirred for 2 hours at room temperature. The precipitated solid was filtered to obtain a crude product, which was a light brown yellow powder in 70% yield.

GC/ MS(m/s) 165 (31) M⁺,148(17), 104(66), 86(100), 72(93), 57(20).

### (3): Synthesis of N¹-((6-chloropyridin-3-yl)methyl)ethane-1,2-diamine

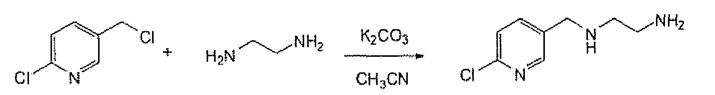

To a stirred solution of 4.2g (0.03mol) of potassium carbonate and 10ml (0.15mol) of ethylenediamine in a 50ml of flask placed in an ice bath, 4.8g (0.03mol) of 2-chloro-5-(chloromethyl)pyridine dissolved in 15ml of' acetonitrile was added dropwise and slowly over nearly 20min. After addition, the ice bath was removed and the mixture was stirred for 8 hours at room temperature. After the reaction was stopped, a large amount of water was added to dissolve potassium carbonate and ethylenediamine, and the mixture was extracted with dichloromethane. The lower organic phase was collected, dried, and evaporated to dryness (It was better to remove all of the ethylenediamine in the solvent since its presence would influence the following step). The obtained product was yellow oily liquid in 68% yield.

GC MS (m/s) 185 (5) M⁺, 155(49), 126(100), 99(9), 90(12).

### (4): Synthesis of 2-chloro-5-((2-(nitromethylene)imidazolidin-1-yl)methyl) pyridine

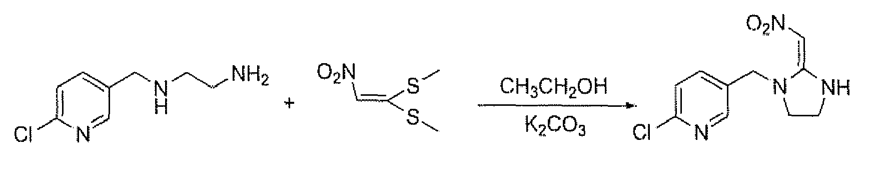

2.5g (0.0178mol) of 1,1-dimethylthio-2-nitroethene, 3.3g (0.0178mol) of N¹-((6-chloropyridin-3-yl)methyl)ethane-1,2-diamine were added to 15ml of ethanol. The resulting mixture was refluxed for 4 hours at 80-90 °C. The mixture was then cooled to educe solid, concentrated, filtrated and dried to give a light yellow powder in 56% yield.

R₁= 0.46 (petroleum ether: ethyl acetate = 1:1).

Mp=156.9°-161.8° GC MS (m/s) 220 (25), 126(100), 90(9).

### (5): Synthesis of 1-((6-chloropyridin-3-yl)methyl)-7-methyl-8-nitro-1,2,3,5,6,7-hexahydroimidazo[1,2-a]pyridin-5-ol (compound 1)

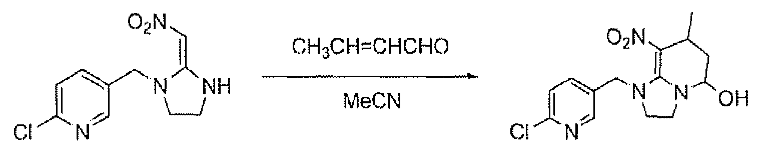

A mixture of 10.16g (0.04mol) of 2-chloro-5-((2-(nitromethylene) imidazolidin-1-yl)methyl)pyridine, 100ml of anhydrous acetonitrile, 5ml of crotonaldehyde and about 4ml of acetic acid in a 250 ml of round-bottomed flask was stirred at 40 °C-45 °C. After about 1 day, a lot of solid precipitated out. The heating was stopped, and the reaction mixture was cooled and filtrated to give a crude product. After recrystallization with MeCN, the pure product was obtained as a yellow powder in 67% yield.

mp = 175.6-177.1 °C;

¹H NMR (500 Mz, DMSO-*d*₆): δ 8.32(d, J=2.14Hz, 1H, pyridine-H), 7.78(dd, J1=2.37Hz, J2=8.21Hz, 1H, pyridine-H), 7.48(d, J=8.22Hz, 1H, pyridine-H), 4.80(m, 1H, -CHOH), 4.64(dd, J1=J2=15Hz, 2H, -CH2-N-), 3.64(m, 4H, imidazolidine-H), 1.91(m, 2H, -CH2CH-), 1.71(m, 1H, -CHCH2-),1.01(d, 3H, -CH3)

HR-MS (EI, 1.08e.3) calcd for C₁₄H₁₇N₄O₃Cl (M⁺), 324.0989; found, 324.0986;
m/z (%)=324(0.75),306(4),294(10),291(100),244(33),126(72);

Anal. Calcd for C₁₄H₁₇ClN₄O₃: C, 51.78; H, 5.28; N, 17.25, Found: C, 51.56; H, 5.17; N, 17.09.

### EXAMPLE 2

### Synthesis of 1-((6-chloropyridin-3-yl)methyl)-5-methoxy-7-methyl-8-nitro-1,2, 3,5,6,7-hexahydroimidazo[1,2-a]pyridine (compound 2)

### (1): Synthesis of 1,1-dichloro-2-nitroethene

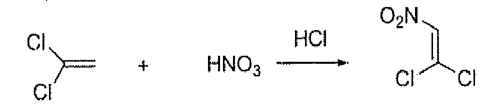

A mixture of 20.85g (0.2055 mol) of 36% hydrochloric acid and 19.9g (0,2055mol) of 65% nitric acid was added to a three-necked flask equipped with a stirring means, and then 15.5g (0.1575 mol) of 1,1-dichloro-2-nitroethene was added dropwise. The addition temperature was controlled between 20 and 25 °C and the mixture was stirred at that temperature for 3h. After completion, the reaction mixture was extracted with 50 ml dichloromethane and the obtained oil layer was washed with water and the PH of the oil layer was adjusted to 3 to 4. 5 to 6g of alkaline was dissolved in 120 ml of water and cooled to 0 °C, then added into the oil layer slowly, and the temperature was kept at 0 °C. After the completion of the addition, the mixture was stirred vigorously for 5 minutes and then extracted with dichloromethane. The layers are separated and the oil layer was washed with water, filtered and dried to give 13.6 to 17.5g of yellow liquid.

### (2): Synthesis of N¹-((6-chloropyridin-3-yl)methyl)ethane-1,2-diamine

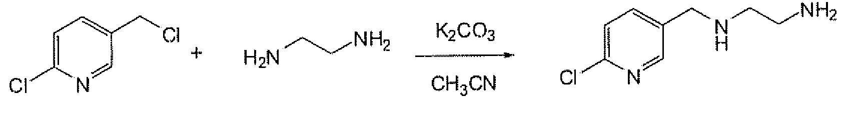

This step was carried out according to step (3) in example 1

### (3): Synthesis of 2-chloro-5-((2-(nitromethylene)imidazolidin-1-yl)methyl) pyridine

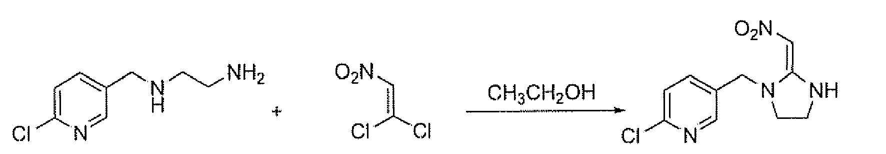

A mixture of 2.53g (0.0178mol) of 1,1-dichloro-2-nitroethene, 3.3g (0.0178mol) or N¹-((6-chloropyridin-3-yl)methyl)ethane-1,2-diamine and 15 ml of ethanol was refluxed at 80 to 90 °C for 4h. The reaction mixture was cooled to educe solid, concentrated, filtrated and dried to give pale yellow power in 56% yield.

R₁ = 0.46 (petroleum ether: ethyl acetate = 1:1).

mp=156.9°C-161.8°C. GC MS (m/s) 220 (25), 126(100), 90(9).

### (4): Synthesis of 1-((6-chloropyridin-3-yl)methyl)-5-methoxy-7-methyl-8-nitro-1,2,3,5,6,7-hexahydroimidazo[1,2-a]pyridine (compound 2)

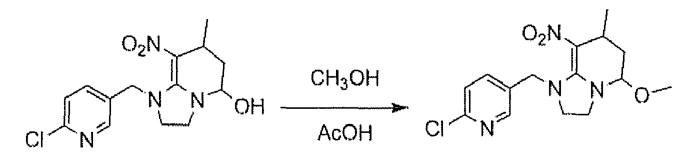

0.324g (0.001mol) of compound 1 was added to a 50ml of three-necked flask, and then 15ml of methanol and a catalytic amount of glacial acetic acid were added. The resulting mixture was refluxed and followed by TLC. After the reaction was complete, the solvent was removed. The residue was then separated by column chromatography to give a pure yellow powder in 60% yield.

mp=164.0-165.4°C;

¹H NMR (500 MHz, CDCl₃): ¹HNMR (500MHz , CDCl₃): 8.32(d, J=2Hz, 1H, pyridine-H), 7.86(dd, J₁=2.4Hz, J₂ =8.22Hz, 1H, pyridine-H), 7.32(d, J=8Hz, 1H, pyridine-H), 4.78(dd, J₁=15Hz, J₂=15Hz, 2H, -CH₂-N-), 4.5(t, J₁=3Hz,J₂=3Hz, 1H, -CHO-), 3.65(m, 4H, imidazolidine-H), 3.36(m, 3H, -OCH₃), 1.87(m, 2H, -CH₂CH-), 1.76(m, 1H, -CHCH₂-); 1.23(d,3H,-CH₃);

HRMS (EI, 2.31e4) calcd for C₁₅H₁₉N₄O₃Cl (M⁺), 338.1146; found, 338.1150;
m/z (%) 338(1), 308(9), 306(1), 291(100), 244(22), 126(38)

Anal. Calcd for C₁₅H₁₉ClN₄O₃: C, 53.18; H, 5.65; N, 16.54, Found: C, 53.35; H, 5.43; N, 17.34.

### EXAMPLE 3

### Synthesis of 1-((6-chloropyridin-3-yl)methyl)-5-ethoxy-7-methyl-8-nitro-1,2,3,5, 6,7-hexahydroimidazo[1,2-a]pyridine (compound 3)

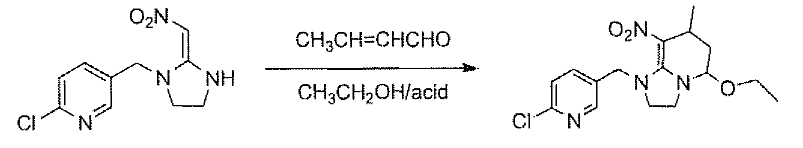

A mixture of 10.16g (0.04mol) of 2-chloro-5-((2-(nitromethylene) -imidazolidin-1-yl)methyl)pyridine, 100ml of anhydrous acetonitrile, about 5ml of crotonaldehyde and a catalytic amount of acetic acid was placed in a 250 ml of round-bottomed flask and refluxed. The reaction was monitored by TLC. After the reaction was complete, the solvent was removed and the residue was separated by column chromatography to give a pure yellow powder in 75% yield.

mp=138.8-140.3 °C;

1H NMR (500 MHz, CDCl₃): δ 8.31(d, J=2Hz, 1H, pyridine-H), 7.87(dd,J₁=2Hz, J₂=8Hz, 1H, pyridine-H), 7.33(d, J=8Hz, 1H, pyridine-H), 4.76(dd, J₁=15Hz, J₂=15Hz, 2H, -CH₂-N-), 4.56 (t, J₁=3Hz, J₂=3Hz, 1H, -CHO-),3.60(m, 4H, imidazolidine-H),3.57(m, 2H, -O-CH₂-), 3.53(m, 1H, -CHCH₂-), 2.01 (m, 2H, -CH₂CH-), 133(t, J₁=7Hz, J₂=7Hz, 3H, -CHCH₃), 1.24(d,3H,-CH₃);

HRMS (EI,7.04e3) calcd for C₁₆H₂₁N₄O₃Cl (M+), 35.2.1302; found, 352.1306;
m/z (%)= 352(3), 337(7), 306(8), 291(100),244(30),126(42)

Anal. Calcd for C₁₆H₂₁ClN₄O₃: C, 54 47; H, 6 00; N, 15.88, Found: C, 54.64; H, 5.96; N, 15.64.

### EXAMPLE 4

### Synthesis of 1-((6-chloropyridin-3-yl)methyl)-5-propyloxy-7-methyl-8-nitro-5-p ropoxy-1,2,3,5,6,7-hexahydroimidazo[1,2-a]pyridine (compound 5)

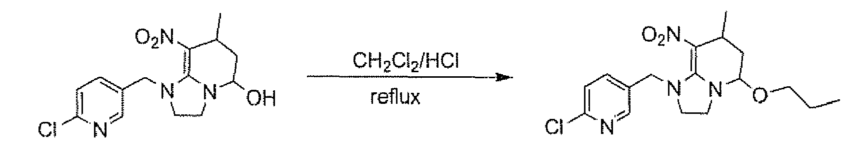

0.972g (0.03mol) of 1-((6-chloropyridin-3-yl)methyl)-7-methyl-8-nitro-1,2,3,5,6,7-hexahydroimidazo[1,2-a]pyridin-5-ol, about 6g of propan-1-ol whose amount was about5 times of that of 1-((6-chloropyridin-3-yl)methyl)-7-methyl-8-nitro-1,2,3,5,6,7-hexahydroimidazo[1,2-a]pyridin-5-ol, 70ml of dichloromethane were added to a 100 ml of three-necked flask and 3 to 4 drops of hydrochloric acid was added to the mixture. Then the mixture was heated under reflux and the reaction was monitored by TLC. After 8 hours, the solvent was removed and the residue was separated by column chromatography, and the column was eluted by 5:1 of petroleum ether and ethyl acetate to wash out propan-1-ol and then eluted by 10:1 of dichloromethane and ethanol to give a pure yellow solid in 72% yield.

mp = 130.2-131.9°C;;

¹H NMR (500MHz , CDCl₃): 8.36(d, J=2Hz, 1H, pyridine-H), 7.79(dd,J₁=2Hz, J₂=8Hz, 1H, pyridine-H), 7.48(d, J=8Hz, 1H, pyridine-H), 4.81(d, J₁=15Hz, J₂=15Hz, 2H, -CH₂-N-), 4.54(t, J₁=3Hz, J₂=3Hz, 1H, -CHO-), 3.77(m, 2H, -O-CH₂-), 3.66(m, 4H, imidazolidine-H), 2.81 (m, 2H, -CH₂CH-), 1.92(m, I H, -CHCH₂-), 1.65(m, 2H, -CH₂CH₃), 1.23(t, J₁=7Hz, J₂=7Hz, 3H, -CHCH₃), 1.03(d,3H,-CH₃).

HRMS (EI,6.83e3) calcd for C₁₇H₂₃N₄O₃Cl (M⁺), 366.1459; found, 366.1487; m/z (%)=366(3), 351(8), 306(4), 291(100), 244(.30), 126(46)

Anal. Calcd for C₁₇H₂₃ClN₄O₃: C, 55.66; H, 6.32; N, 15.27, Found: C, 55.50; H, 6.21; N, 15.08.

### EXAMPLE 5

### Synthesis of 5-butoxy-1-((6-chloropyridin-3-yl)methyl)-7-methyl-8-nitro-1,2,3, 5,6,7-hexahydroimidazo[1,2-a]pyridine (compound 7)

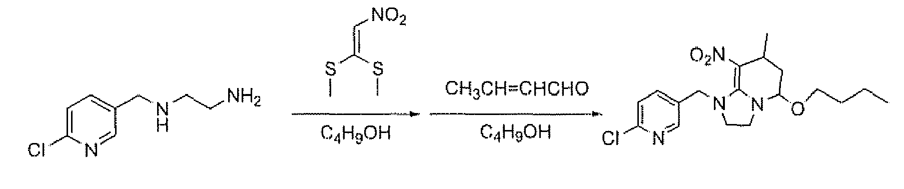

A mixture of 3.713g (0.02mol) of 1,1-bis(methylthio)-2-nitroethene, 3.305g (0.02mol) of N¹-((6-chloropyridin-3-yl)methyl)ethane-1,2-diamine, 15ml of butan-1-ol was refluxed at 80-90 °C for 4h, then 3ml of crotonaldehyde was added to the reaction mixture and the reaction mixture was heated under reflux. The reaction was monitored by TLC. After the reaction was complete, the solvent was removed and the residue was separated by column chromatography to give a pure yellow solid in 75% yield.

mp = 103.5-105.2°C;

¹H NMR (500MHz , CDCl₃): 8.32(d, J=2Hz, 1H, pyridine-H), 7.85(dd,J₁=2Hz, J₂=8Hz, 1H, pyridine-H), 7.33(d, J=8Hz, 1H, pyridine-H), 4.74(d, J₁=15Hz, J₂=15Hz, 2H, -CH₂-N-), 4.49(t, J₁=3Hz, J₂=3Hz, 1H, -CHO-), 3.77(m, 2H, -O-CH₂-), 3.57(m, 4H, imidazolidine-H), 2.86(m, 2H, -CH₂CH₂CH₂CH₃), 2.81 (m, 2H, -CH₂CH-), 1.92(m, 1H, -CHCH₂-), 1.63(m, 2H, -CH₂CH₂CH₃), 1.35(m, 4H, -CH₂CH₃), 1.27(t, J₁=7Hz, J₂=7Hz, 3H, -CHCH₃), 0.92(d,3H,-CH₃);

HRMS (EI,6.83e3) calcd for C₁₇H₂₃N₄O₃Cl (M⁺), 366.1459; found, 366.1487; m/z (%) = 366(3),351(8),306(4), 291(100),244(30),126(46)

Anal. Calcd for C₁₇H₂₃ClN₄O₃: C, 56.76; H, 6.62; N, 14.71, Found: C, 56.68; H, 6.41; N, 14.52.

### EXAMPLE 6

### Synthesis of 1-((6-Chloropyridin-3-yl)methyl)-7-methyl-8-nitro-1,2,3,5,6,7-hexa hydroimidazo[1,2-a]pyridin-5-yl acetate (compound 11)

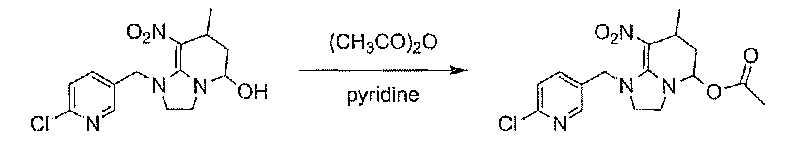

0,001mol of compound I was added to a 50ml of rouned-bottomed flask and then 0.001mol of acetic anhydride and 0.001mol of pyridine were added and the resulting mixture was stirred at room temperature and the reaction was monitored by TLC. After the reaction was complete, the solvent was removed and the residue was separated by column chromatography to give a pure pale yellow powder in 70% yield.

mp = 144-145.7 °C;

IR (KBr cm⁻¹) 2903, 2370, 1713, 1332, 1203, 1123, 1071, 1000, 973, 830, 592;

¹H NMR (500MHz , CDCl₃): 8.33(d, J=2Hz, 1H, pyridine-H), 7.88(dd, J₁=2Hz, J₂ =8Hz, 1H, pyridine-H), 7.36(d, J=8Hz, I H, pyridine-H), 4.79(d, J₁=15Hz, J₂=15Hz, 2H, -CH₂-N-), 4.48(t, J₁=3Hz,J₂=3Hz, 1H, -CHO-), 4.12(m, 3H, -OCOCH₃), 3.62(m, 4H, imidazolidine-H), 2.97(m, 2H, -CH₂CH₂-), 1.87(m, 2H, -CH₂CH-), 1.76(m, 1H, -CHCH₂-);

HRMS (EI,2.08e3) calcd for C₁₅H₁₉N₄O₄Cl (M⁺), 366.1095; found, 366 1093;
m/z (%)=366(1), 351(11), 336(8), 306(19), 291(100), 244(22), 126(38)

Anal. Calcd for C₁₅H₁₉ClN₄O₄: C, 52.39; H, 5.52; N, 15.27, Found: C, 52.23; H, 4,98; N, 15.02.

### EXAMPLE 7

### Synthesis of 1-((2-chlorothiazol-5-yl)methyl)-7-methyl-8-nitro-1,2,3,5,6,7-hexa hydroimidazo[1,2-a]pyridin-5-ol (compound 12)

### (1): Synthesis of N¹-((2-chlorothiazol-5-yl)methyl)ethane-1,2-diamine

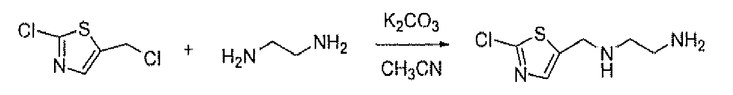

To a stirred solution of 0.03mol of potassium carbonate and 10ml (0.15mol) of ethylenediamine in a 50ml of flask placed in an ice bath, 0.03mol of 2-chloro-5-(chloromethyl)thiazole dissolved in 15ml of acetonitrile was added dropwise and slowly. After addition, the ice bath was removed and then the mixture was stirred for 8 hours at room temperature. After the reaction was complete, a large amount of water was added to dissolve potassium carbonate and ethylenediamine, and the mixture was extracted with dichloromethane. The lower organic phase was collected, dried, and evaporated to dryness. The obtained product was yellow oily liquid in 70% yield.

GC MS (m/s) 191 (21) M⁺, 132(100).

### (2): Synthesis of 1-((2-chlorothiazol-5-yl)methyl)-2-(nitromethylene)-1-imidazolidine

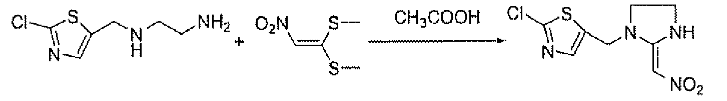

2.5g (0.0178mol) of 1,1-dimethylthio-2-nitroethene, 3.3g (0.0178mol) of N¹-((2-chlorothiazol-5-yl)methyl)ethane-1,2-diamine were dissolved in 15ml of ethanol. The resulting mixture was refluxed at 80-90 °C for 4 hours. The mixture was then cooled to educe solid, concentrated, filtrated and dried to give a light yellow powder in 56% yield.

GC MS(m/s) 226 (24), 132(100), 77(9).

### (3): Synthesis of 1-((2-chlorothiazol-5-yl)methyl)-7-methyl-8-nitro-1,2,3,5,6,7-hexahydroimidazo[1,2-a]pyridin-5-ol

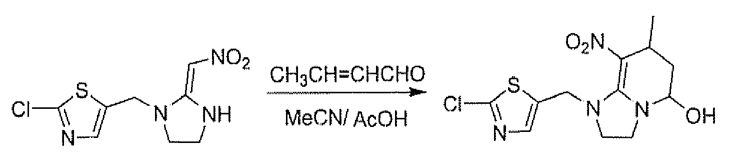

0.002mol of 1-((2-chlorothiazol-5-yl)methyl)-2-(nitromethylene) imidazolidine, 15ml of anhydrous acetonitrile, 0.25ml of crotonaldehyde and a catalytic amount of acetic acid were placed in a 50ml of round-bottomed flask and stirred at 40-45 °C. After about 1 day, a lot of solid precipitated out. The heating was stopped, and the reaction mixture was cooled and filtrated to give a crude product. After recrystallization with MeCN, the pure product was obtained as a yellow powder in 40% yield.

mp=172-174.6°C;

IR (KBr, cm⁻¹) 3194, 1564, 1545, 1373, 1310, 1150;

¹H NMR (500MHz ,DMSO): 7.38(s, 1H, thiazole-H), 4.83(m, 1H, -CHOH), 4.59(dd, J₁=J₂=15Hz, 2H, -CH₂-N-), 3.56(m, 4H, imidazolidine-H), 1.93(m, 2H, -CH₂CH-), 1.75(m, 1H, -CHCH₂-),1.13(d, 3H, -CH₃);

HRMS (E1,3.38e3) calcd for C₁₂H₁₅N₄O₃ClS (M⁺), 330.0553; found, 330.0786;
m/z(%)=330(2), 315(4), 312(10), 297(100), 132(72) Anal. Calcd for C₁₂H₁₅N₄O₃ClS: C, 43.57; H, 4.57; N, 16.94, Found: C, 43.39; H, 4.35; N, 16.65.

### EXAMPLE 8

### Synthesis of 1-((6-chloropyridin-3-yl)methyl)-6-methyl-8-nitro-1,2,3,5,6,7-hexa hydroimidazo[1,2-a]pyridin-5-ol (compound 18)

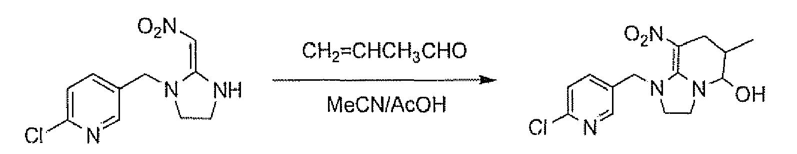

A mixture of 0.509g (0.002mol) of 2-chloro-5-((2-(nitromethylene) -imidazolidin-1-yl)methyl)pyridine, 25ml of anhydrous acetonitrile, 0.20ml of methacrylaldehyde and a catalytic amount of acetic acid (If appropriate, acetic acid may be added more) in a 50 ml of round-bottomed flask was stirred at room temperature About four days later, a lot of solid precipitated out (during the reaction, a proper amount of methacrylaldehyde may be supplemented). The reaction was stopped, and the precipitated solid was filtrated to give a crude product. After recrystallization, the pure product was obtained as a milk white powder in 63% yield.

mp = 179.6-181.1 °C;

¹H NMR (500MHz ,DMSO): 8.32(d, J=2.14Hz, 1H, pyridine-H), 7.78(dd, J₁=2.37Hz, J₂=8.21Hz, 1H, pyridine-H), 7.48(d, J=8.22Hz, 1H, pyridine-H), 4.80(m, 1H, -CHOH), 4.64(dd, J₁=J₂=15Hz, 2H, -CH₂-N-), 3.64(m, 4H, imidazolidine-H), 1.99(m, 2H, -CHCH₂-), 1,78(m, 1H, -CH₂CH-),1.05(d, 3H, -CH₃);

HRMS (EI,1.08e3) calcd for C₁₄H₁₇N₄O₃Cl (M⁺), 324.0989; found, 324.0982;
m/z (%)=324(2), 306(8), 294(10), 291(100), 244(43),126(70)

Anal Calcd for C₁₄H₁₇N₄O₃C1: C, 51.78; H, 5.28; N, 17.25, Found: C, 51.63; H, 5.11; N, 17.02.

### EXAMPLE 9

### Synthesis of 1-((6-chloropyridin-3-yl)methyl)-6,7-dimethyl-8-nitro-1,2,3,5,6,7-h exahydroimidazo[1,2-a]pyridin-5-ol (compound 30)

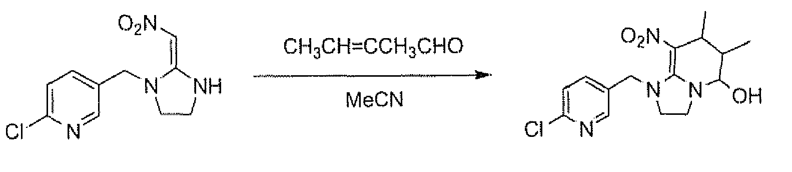

A mixture of 0.509g (0.002mol) of 2-chloro-5-((2-(nitromethylene)imidazolidin-1-yl)methyl)pyridine, 25ml of anhydrous acetonitrile, 0.20ml of 2-methyl but-2-enal and a catalytic amount of acetic acid (If appropriate, acetic acid may be added more) in a 50 ml of round-bottomed flask was stirred at room temperature. About four days later, a lot of solid precipitated out (during the reaction, a proper amount of 2-methyl but-2-enal may be supplemented). The reaction was stopped, and the precipitated solid was filtrated to give a crude product. After recrystallization, the pure product was obtained as a milk white powder in 60% yield.

mp= 168.6-171.1 °C;

¹H NMR (500MHz ,DMSO): 8.34(d, J=2.14Hz, 1H, pyridine-H), 7.81(dd, J₁=2.37Hz, J₂=8.21Hz, 1H, pyridine-H), 7,51(d, J=8.22Hz, 1H, pyridine-H), 4.79(m, 1H, -CHOH), 4.61(dd, J₁=J₂= 15Hz, 2H, -CH₂-N-), 3.67(m, 4H, imidazolidine-H), 1. 96(m, 1H, -CHCH-), 1.51(m, 1H, -CHCH-),1.19(d, 3H, -CH₃), 1.01(d, 3H, -CH₃);

HRMS (EI,4.33e4) calcd for C₁₅H₁₉N₄O₃Cl (M⁺), 338.1146; found, 338.1140;
m/z (%)=338(2),320(7),323(23),308(3),291(100),244(22),126(38);

Anal. Calcd for C₁₅H₁₉N₄O₃Cl: C, 53.18; H, 5.65; N, 16.54, Found: C, 52.95; H, 5.41; N, 17.30.

### EXAMPLE 10

### Synthesis of 1-((6-chloropyridin-3-yl)methyl)-8-nitro-7-phenyl-1,2,3,5,6,7-hexa hydroimidazo[1,2-a]pyridin-5-ol (compound .38)

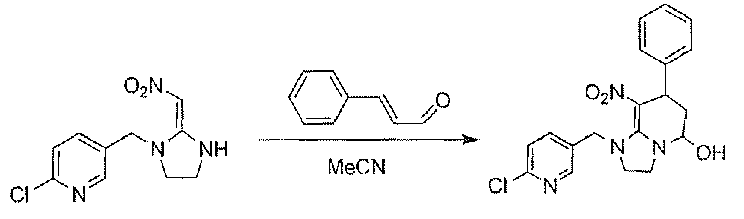

A mixture of 0.509g (0.002mol) of 2-chloro-5-((2-(nitromethylene) -imidazolidin-1-yl)methyl)pyridine, 25ml of anhydrous acetonitrile, 0.20ml of cinnamaldehyde and a catalytic amount of acetic acid (If appropriate, acetic acid may be added more) in a 50 ml of round-bottomed flask was stirred at room temperature. About four days later, a lot of solid precipitated out (during the reaction, a proper amount of cinnamaldehyde may be supplemented). The reaction was stopped, and the precipitated solid was filtrated to give a crude product. After recrystallization, the pure product was obtained as a milk white powder in 40% yield.

mp = 229.3-230.1°C;

HNMR (500MHz, DMSO): 8.39(d, J=2.14Hz, 1H, pyridine-H), 7.87(dd, J₁=2.37Hz, J₂=8.21Hz, 1H, pyridine-H), 7.32(d, J=8.2Hz, 1H, pyridine-H), 7.24(d, J=7.64Hz,2H,benzene-H),7.17(t,1H, benzene-H ), 7.13(d, J=7.22Hz,2H,benzene-H), 4.83(dd, J₁=J₂=15Hz, 2H, -CH₂-N-), 4.56(m, 1H, -CHOH), 4.44(t, 1H, -CHCH₂-), 3.66(m, 4H, imidazolidine-H),2.33(m, 2H, -CH₂CH-);

HRMS (EI,5.96e3) calcd for C₁₉H₁₉N₄O₃Cl (M⁺), 386.1146; found, 386.1121;
m/z (%)=386(2), 320(7), 340(7), 210(10), 208(21), 126(100);

Anal. Calcd for C₁₉H₁₉N₄O₃Cl: C, 58.99; H, 4.95; N, 14.48, Found: C, 58.76; H, 4.87; N, 14.33.

### EXAMPLE 11

### Synthesis of 1-((6-chloropyridin-3-yl)methyl)-7-methoxy-8-nitro-1,2,3,5,6,7-he xahydroimidazo[1,2-a]pyridin-5-ol (compound 42)

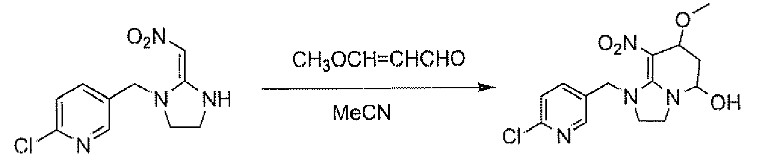

A mixture of 10.16g (0.04mol) of 2-chloro-5-((2-(nitromethylene)imidazalidin-1-yl)methyl)pyridine, 100ml of anhydrous acetonitrile, about 5ml of 3-methoxyacrylaldehyde and about 4ml of acetic acid (1f appropriate, acetic acid may be added more) in a 250 ml of round-bottomed flask was stirred at 40°C-45°C. About 1 day later, a lot of solid precipitated out. The reaction was stopped and cooled, and the precipitated solid was filtrated to give a crude product. After recrystallization from MeCN, the pure product was obtained as a yellow powder in 70% yield.

¹H NMR (500MHz ,DMSO): 8.34(d, J=2.14Hz, 1H, pyridine-H), 7.79(dd, J ₁=2.37Hz, J₂=8.21Hz, 1H, pyridine-H), 7.50(d, J=8.22Hz, 1H, pyridine-H), 5.05 (t, 1H, -CHCH₂-),4.95(m, 1H, -CHOH), 4.84(dd, J₁=J₂=15Hz, 2H, -CH₂-N-), 3.9 7(s,3H,-OCH₃),3.76(m, 4H, imidazolidine-H), 2.51(m, 2H, -CH₂CH-);

HRMS (EI,2.63e3) calcd for C₁₄H₁₇N₄O₄Cl (M⁺), 340.0938; found, 340.0936;
m/z (%)=340(3),322(4),308(11),291(100),244(37),126(78);

Anal. Calcd for C₁₄H₁₇N₄O₄Cl: C, 49.35; H, 5.03; N, 16.44, Found: C, 48.76; H, 4.87; N, 16.33.

### EXAMPLE 12

### Synthesis of 1-((6-chloropyridin-3-yl)methyl)-6-methoxy-8-nitro-1,2,3,5,6,7-he xahydroimidazo[1,2-a]pyridin-5-ol (compound 42)

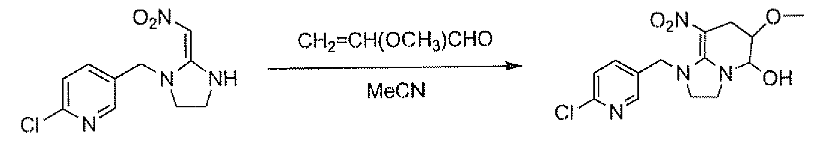

A mixture of 10.16g (0.04mol) of 2-chloro-5-((2-(nitromethylene) -imidazolidin-1-yl)methyl)pyridine, 100ml of anhydrous acetonitrile, 5ml of 2-methoxyacrylaldehyde and about 4ml of acetic acid (If appropriate, acetic acid may be added more) in a 250 ml of round-bottomed flask was stirred at 40°C-45°C. About 1 day later, a lot of solid precipitated out. The reaction was stopped and cooled, and the precipitated solid was filtrated to give a crude product. After recrystallization, the pure product was obtained as a yellow powder in 75% yield.

¹H NMR (500MHz ,DMSO): 8.34(d, J=2.14Hz, 1H, pyridine-H), 7.79(dd, J₁=2.37Hz, J₂₌8.21Hz, 1H, pyridine-H), 7.50(d, J=8.22Hz, 1 H, pyridine-H), 5.05(t, 1H, -CHCH₂-),4.95(m, 1H, -CHOH), 4.84(dd, J₁=J₂=15Hz, 2H, -CH₂-N-), 3.93(s,3H,-OCH₃), 3.76(m, 4H, imidazolidine-H), 2.49(m, 2H, -CH₂CH-);

HR-MS (EI,2.63e3): calcd for C₁₄H₁₇N₄O₄Cl (M+): 340.0938, found:340.0936, m/z (%)=340(3), 322(4), 308(11), 291(100), 244(37), 126(78)

Anal. Calcd for C₁₄H₁₇N₄O₄Cl: C, 49.35; H, 5.03; N, 16.44, Found: C, 48. 76; H, 4 87; N, 16.33.

The compounds of the present invention can be used to control and kill general insects, including sucking insects, biting insects and other plant parasites, storage cereal insects and health hazard insects.

The examples of insects are listed as follow:
Coleoptera: *Sitophilus zeamais*, *Tribo*/*ium castaneum*, *Henosepilacha vigintioctomaculata*, *Agriotes fuscicollis*, *Monolepta hieroglyphica*, *Diabrotica SPP*, *Anomala cupripes*, *Monochamus alternatus, Echinocnemus squameus*, *Echinocnemus bipunctaus*, *Lissorhoptrus oryzophilus*, *Lyrtus hrunneus;*
Lepidoptera: *Lymantria dispar*, *Malacosoma neustria testacea*, *Prodenia litura*, *Mamestra brassicae*, *Chilo suppressalis*, *Ostrinia nubilalis*, *Cadra cautella*, *Adoxophyes orana, Laspeyresia splendana*, *Agrotis fucosa*, *Galleria mellonella*, *Plutella xylostella*, *Phyllocnistis citrella*,
Hemiptera: *Nephotettix cincticeps*, *Nilaparvata lugens*, *Laodelphax striatellus*, *Bemisia tabaci*, *Pseudococcus comstocki*, *Unaspis yanonensis*, *Myzus persicae*, *Aphis pomi*, *Brevicoryne brassicae*, *Lipaphis erysimi pseudobrassicae*, *Stephanitis nashi*, *Nazara SPP*., *Cimicidae*, *Trialeurodes vaporariorum*, and *Psylle SPP*;
Orthoptera: *Blattella germanica*, *Periplaneta americana*, *Gryllotalpa africana*, *Locusta migratoria*.
Isoptera: *Deucotermes speratus*, *Coptotermes formosanus*,
Diptera: *Musca domestica*, *Aedes aegypti*, *Hylemya platura*, *Delia platura*, *Anopheles sinensis* and *Tetranychus cinnabarinus*.

The compounds in this invention have special effects to insects having a piercing-sucking or scratching monthparts, such as aphid, leafhopper, plant hopper, thrips, white fly.

These active compounds can be prepared into the customary formulations, such as solutions, emulsions, suspensions, powders, foams, pastes, granules, aerosols, especially soluble solid and liquid preparations which are compatible with the environment such as water-dispersed granules, water solution and water emulsion, ultra low volume preparations, natural and synthetic materials impregnated with active compounds, and micro-capsules in polymers used in the coating complex for seed, preparations used with a combustion device (such as smoking cylindrantherae, smoking can and smoking plate) and ULV cold mist and warm mist preparations. These formulations may be produced in a known manner, for example, by mixing the active compounds with extenders, which are liquid or liquefied gas or solid diluents or carriers, optionally with the use of surface-active agents, i.e. emulsifying agents and/or dispersing agents, and/or foam-forming agents. In the case of using water as an extender, organic solvents can, for example, also be used as auxiliary solvents.

It is generally proper to use liquid solvents as a diluent or carrier, for example, aromatic hydrocarbons, such as xylene, toluene and alkyl naphthalenes, chlorinated aromatic or chlorinated aliphatic hydrocarbons, such as chlorobenzenes, chloroethylenes and methylene chloride, aliphatic hydrocarbons, such as cyclohexane or paraffins, for example, mineral oil fractions; alcohols, such as butanol or glycol as well as their ethers and esters; ketones, such as acetone, methyl ethyl ketone, methyl isobutyl ketone or cyclohexanone, or uncommon polar solvents, such as dimethylformamide and dimethylsulfoxide, as well as water.

By liquefied gas diluents or carriers are meant liquids which are gaseous at normal temperature and under normal pressure, for example, aerosol propellants, such as halogenated hydrocarbons as well as butane, propane, nitrogen and carbon dioxide

The solid carrier can use ground natural minerals, such as kaolins, clays, talcs, quartzs, attapulgites, montmorillonites or kieselguhrs; ground synthetic minerals, such as high dispersed silicic acid, alumina and silicate. The solid carrier used for particles is crushed and fractionated natural rocks such as calcite, marble, pumice, sepiolite and dolomite, as well as synthetic granules of inorganic and organic coarse powder, and granules of organic material such as sawdust, coconut shells, maize cobs and tobacco stalks and the like.

Nonionic and anionic emulsifiers may be used as emulsifying and/or foam-forming agents, such as polyoxyethylene-fatty acid esters, polyoxyethylene-fatty alcohol ethers, for example, alkylaryl polyglycol ethers, alkylsulfonates, alkylsulfates, arylsulfonates as well as albumin hydrolysis products. Dispersing agents include, for example, lignin sulfite waste liquors and methyl cellulose.

Adhesives such as carboxymethyl cellulose and natural and synthetic polymers, (such as gum arabic, polyvinyl alcohol and polyvinyl acetate) in the form of powders, granules or emulsions can be used in the formulations

It is possible to use colorants such as inorganic dyestuffs, for example, iron oxide, cobalt oxide and Prussian Blue, and organic dyestuffs, such as diazo dyestuffs or metal phthalocyanine dyestuffs, and trace nutritional agent, such as the salts of iron, manganese, boron, copper, cobalt, aluminum, zinc and the like

The formulations, in general, contain from 0.1 to 95 percent by weight of active compound, preferably 0.5-90 percent by weight.

The active compound of the present invention can be present as a mixture with other active compounds in a commercial formulation or a use form prepared from the commercial formulation. The other active compounds can be insecticide, bait formulation, bactericide, acaricide, nematocide, fungicide, growth controller and the like. The insecticide includes phosphates, carbamates, pyrethroids, chlorinated hydrocarbons, benzoylurea, nereistoxin and material produced by microbion such as avermectin.

Furthermore, the active compound of the present invention can be present as a mixture with a synergist in a commercial formulation or a use form prepared from the commercial formulation Synergist is used to enhance the action of active compound, so if the compound itself is active there is no need to use it The concentration of the active compound in the use form prepared from the commercial formulation can vary within a wide range. The active compound concentration of the formulation for use is, for example, from 0.0000001 to 100 percent by weight of active compound, preferably from 0.0001 to 1 percent by weight.

These compounds can be prepared into proper dosage forms and used by common methods.

### ACTIVITY TEST

### Test on the insecticidal activity of compounds of the present invention

Aphis, which belongs to Homoptera and has a piercing-sucking mouthpart, is a common insect for agricultural plant. *Aphis craccivora* was tested by the way of immersing.

Test method: exactly weighed various samples were independently added to N,N-dimethylformamide to form a 10g/L stock solution. The mixture was diluted with 0.2mL/L aqueous Triton X-100 solution to a concentration of 500ug/mL. After stably sucking on bean sprout, the adult aphis without wings together with bean sprout was dipped into the dilution of 500 ug/mL, 100 ug/mL, 50 ug/mL, 25 ug/mL, 12.5 ug/mL, 6.25 ug/mL (LD₅₀ was calculated), taken out after 5 seconds, and the excess dilution was sucked out with bibulous paper and the adult aphis without wings was incubated in clean vessel at a constant temperature of 23 °C. Each concentration was repeated for 3 times and the control group contained 0.2mL/L, aqueous Triton X-100 solution. The number of killed aphis was counted after 24 hours to calculate the mortality. The result was shown in Table 1 bellow

Plant hopper, which belongs to Homoptera and has a piercing-sucking mouthpart, is a common insect for agricultural plant. *Nilaparvata lugens* was tested by the way of spraying.

Test method: the compound to be tested was exactly formulated into a solution having a certain concentration and clean water was used as a blank control. Each process was repeated for 3 tumblers (3 times). 2ml of solution was sprayed uniformly to each tumbler by a mini manual sprayer. 10 *Nilaparvata lugens* were introduced to every sink 6 hours before spraying. Three series of experiments were conducted. The number of killed *Nilaparvata lugens* was counted after 24 hours to calculate the mortality. The result was shown in Table 1 and Table 2 bellow.

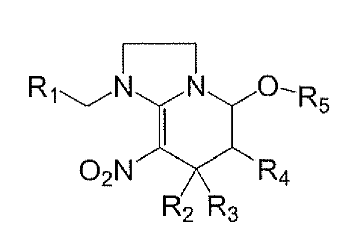

**Table 1**

| Number | R₁ | R₂ | R₃ | R₄ | R₅ | Mortality (%) (500ppm) | |
|---|---|---|---|---|---|---|---|
| | | | | | | Aphis craccivora | *Nilaparvata lugens* |
| 1 | 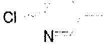 | CH₃ | H | H | H | 100 | 100 |
| 2 | 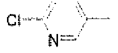 | CH₃ | H | H | CH₃ | 98 | 100 |
| 3 | 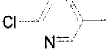 | CH₃ | H | H | CH₂CH₃ | 100 | 100 |
| 4 | 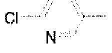 | CH₃ | H | H | CH₂CF₃ | 100 | 100 |
| 5 | 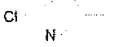 | CH₃ | H | H | CH₂CH₂CH₃ | 98 | 100 |
| 6 | 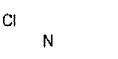 | CH₃ | H | H | CH(CH₃)₂ | 100 | 100 |
| 7 | 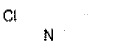 | CH₃ | H | H | CH₂(CH₂)₂CH₃ | 80 | 100 |
| 8 | 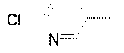 | CH₃ | H | H | CH₂(CH₂)₃CH₃ | 67 | 80 |
| 9 | 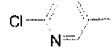 | CH₃ | H | H | CH₂CH=CH₂ | 98 | 100 |
| 10 | 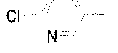 | CH₃ | H | H | CH₂C≡CH | 43 | 67 |
| 11 | 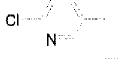 | CH₃ | H | H | COCH₃ | 93 | 100 |
| 12 | 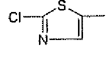 | CH₃ | H | H | H | 100 | 100 |
| 13 | 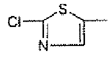 | CH₃ | H | H | CH₂CH₃ | 98 | 100 |
| 14 | 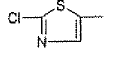 | CH₃ | H | H | CH₂CH₂CH₃ | 100 | 100 |
| 15 | 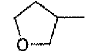 | CH₃ | H | H | H | 24 | 35 |
| 16 | 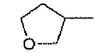 | CH₃ | H | H | CH₃ | 45 | 67 |
| 17 | 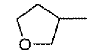 | CH₃ | H | H | CH₂CH₃ | 89 | 100 |
| 18 | 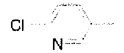 | H | H | CH₃ | H | 98 | 100 |
| 19 | 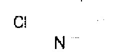 | H | H | CH₃ | CH₂CH₃ | 100 | 100 |
| 20 | 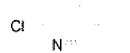 | H | H | CH₃ | CH₂CH₂CH₃ | 86 | 98 |
| 21 | 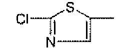 | H | H | CH₃ | H | 90 | 100 |
| 22 | 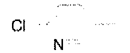 | CH₂CH₂ | H | H | H | 93 | 98 |
| 23 | 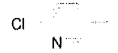 | CH₂CH₂ | H | H | CH(CH₃)₂ | 65 | 95 |
| 24 | 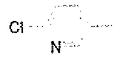 | CH₂CH₂CH₃ | H | H | H | 97 | 89 |
| 25 | 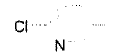 | CH₂CH₂CH₃ | H | H | CH₂CH₃ | 96 | 100 |
| 26 | 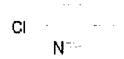 | H | H | CH₂CH₃ | H | 100 | 100 |
| 27 | 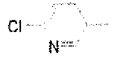 | H | H | CH₂CH₃ | CH₃ | 92 | 80 |
| 28 | 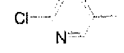 | H | H | CH₂CH₂CH₃ | H | 100 | 96 |
| 29 | 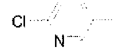 | H | H | CH₂CH₂CH₃ | CH₂CH₃ | 100 | 97 |
| 30 | 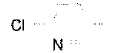 | CH₃ | H | CH₃ | H | 100 | 100 |
| 31 | 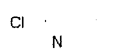 | CH₃ | CH₃ | H | H | 96 | 100 |
| 32 | 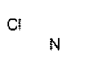 | CH₃ | CH₃ | H | CH₃ | 100 | 100 |
| 33 | 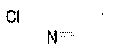 | CH₃ | H | CH₂CH₃ | H | 92 | 78 |
| 34 | 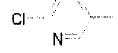 | CH₃ | H | CH₂CH₂CH₃ | H | 95 | 90 |
| 35 | 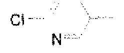 | CH₂CH₃ | H | CH₃ | H | 100 | 88 |
| 36 | 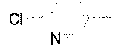 | CH₃ CH₃ | CH₃ | CH₃ | H | 94 | 97 |
| 37 | 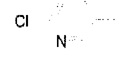 | CH₃ CH₃ | CH₃ | CH₂CH₃ | H | 99 | 88 |
| 38 | 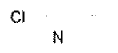 | 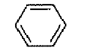 | H | H | H | 24 | 0 |
| 39 | 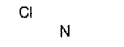 | 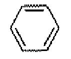 | H | H | CH₃ | 45 | 0 |
| 40 | 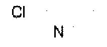 | CH(CH₃) | H | H | H | 87 | 56 |
| 41 | 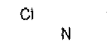 | n-C₄H₉ | H | H | H | 72 | 50 |
| 42 | 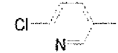 | OCH₃ | H | H | H | 100 | 97 |
| 43 | 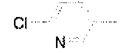 | OCH₃ | H | CH₃ | H | 100 | 97 |
| 44 | 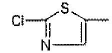 | OCH₃ | H | H | H | 100 | 97 |
| 45 | 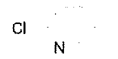 | OCH₂CH₃ | H | H | H | 100 | 89 |
| 46 | 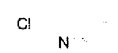 | H | H | OCH₃ | H | 100 | 73 |
| 47 | 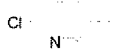 | H | H | OCH₂CH₃ | H | 98 | 91 |
| 48 | 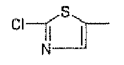 | H | H | OCH₃ | H | 85 | 90 |
| 49 | 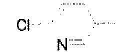 | CH₃ | H | OCH₃ | H | 100 | 100 |

**Table 2**

| Number | R₁ | R₂ | R₃ | R₄ | R₅ | Aphis craccivora | |
|---|---|---|---|---|---|---|---|
| | | | | | | Mortality(%) (100ppm) | LC₅₀ (ppm) |
| a | 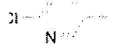 | CH₃ | H | H | H | 95.3 | 31.5 |
| b | 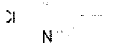 | CH₃ | H | H | CH₃ | 84 | 50.8 |
| c | 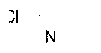 | CH₃ | H | H | CH₂CH₃ | 67 | 65.4 |
| d | 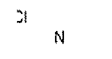 | CH₃ | H | H | CH₂CH₂CH₃ | 75.4 | 33.6 |
| e | 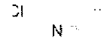 | CH₃ | H | H | CH(CH₃)₂ | 100 | 20.5 |
| f | 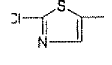 | CH₃ | H | H | H | 87 | 68 |
| g | 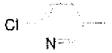 | H | H | CH₃ | H | 92 | 36.3 |
| h | 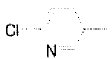 | CH₂CH₂ | H | H | H | 83 | 43.9 |
| i | 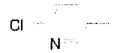 | CH₂CH₂CH₃ | H | H | H | 77 | 75.2 |
| j | 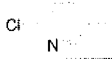 | H | H | CH₂CH₃ | H | 93.6 | 57.3 |
| k | 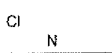 | H | H | CH₂CH₃ | CH₃ | 92 | 83.0 |
| l | 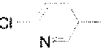 | CH₃ | H | CH₃ | H | 66.7 | 86.4 |
| m | 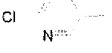 | CH₂CH₃ | H | CH₃ | H | 87 | 75.9 |
| n | 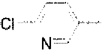 | OCH₃ | H | H | H | 96 | 40.5 |
| o | 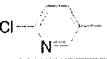 | OCH₂CH₃ | H | H | H | 82 | 66.4 |
| p | 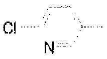 | H | H | OCH₃ | H | 93 | 47.6 |
| q | 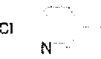 | H | H | OCH₂CH₃ | H | 85 | 70.9 |

### COMPOSITION EXAMPLE 1

### (a) Oily Suspension

| | |
|---|---|
| Any one of compounds a-q in Table 2 | 25 wt% 25 wt% |
| Polyoxyethylene sorbitol hexaoleate | 5 wt% |
| Higher aliphatic hydrocarbon oil | 70 wt% |

Each of the components was ground in a sand mill until the solid granules were reduced to less than about 5 micrometer. The resulting viscous suspension can be used directly or may be used after it was emulsified in water.

### (b) Aqueous Suspension

| | |
|---|---|
| Any one of compounds a-q | 25 wt% |
| Hydrate attapulagit | 3 wt% |
| Calcium lignosulphonate | 10 wt% |
| Sodium dihydrogen phosphate | 0.5 wt% |
| Water | 61.5 wt% |

Each of the components was ground in a ball mill until the solid granules were reduced to less than about 10 micrometer. The aqueous suspension can be used directly.

### (c) bait formulation

An edible bait was prepared with the components as follows:

| | |
|---|---|
| Any one of compounds a-q | 0.1-10 wt% |
| Wheat flour | 80 wt% |
| Molasses | 19.9-10 wt% |

These ingredients were intimately mixed and forged as required into a bait form. This edible bait may be distributed at a locus, for example domestic or industrial premises, e. g. kitchens, hospitals or stores, or outdoor areas, infested by the sanitary insects, to control the insects by oral ingestion.

### (d) wettable power

A wettable powder was prepared with the components as follows:

| | |
|---|---|
| Any one of compounds a-q | 30 wt% |
| sodium dodecyl benzene sulfonate | 2 wt% |
| Sodium lignosulphonate | 5 wt% |
| Synthetic magnesium silicate support | 63wt% |

Each of these components was ground in a hammer-mill until the solid granules were reduced to less than about 50 micrometer to give a wettable powder, which may be applied to the plant seeds or plant leaves and/or plant fruits or the place where the plant is growing or is expected to be grown by dipping, or by oral administration in drinking water, to prevent the insects.

All the documents cited herein are incorporated into the invention as reference, as if each of them is individually incorporated. Further, it would be appreciated that, in light of the above described teaching of the invention, the skilled in the art could make various changes or modifications to the invention, and these equivalents would still be within the scope of the invention defined by the appended claims of the application.

## Claims

1. A nitromethylene derivative represented by formula (I), 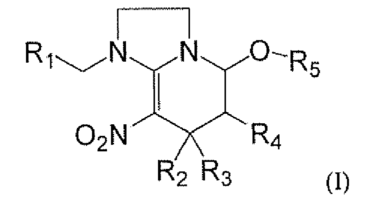 wherein
R₁ is a 5 or 6-membered heterocycle containing nitrogen, oxygen and/or sulfur, or a halogenated 5 or 6-membered heterocycle containing nitrogen, oxygen and/or sulfur;
R₂ is a hydrogen atom, C₁₋₄ alkyl group, C₁₋₄ alkoxyl group or aryl group;
R₃ is a hydrogen atom, C₁₋₄ alkyl group, or C₁₋₄ alkoxyl group;
R₄ is a hydrogen atom, C₁₋₄ alkyl group, C₁₋₄ alkoxyl group or aryl group;
R₅ is a hydrogen atom, a saturated or unsaturated C₁₋₈ hydrocarbyl group, saturated or unsaturated C₁₋₈ halogenated hydrocarbyl group, -CH₂CH₂OCH₂CH₃, -CH₂CH₂OCH₃, saturated or unsaturated C₁₋₈ hydrocarbyloxy,

2. The derivative according to claim 1, wherein R₁ represents one of pyridyl, thiazolyl, and tetrahydrofuryl, or the chloride thereof.

3. The derivative according to claim 2, wherein R₁ represents 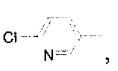 or

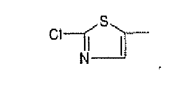

4. The derivative according to claim 1, wherein R₅ represents a hydrogen atom or saturated or unsaturated C₁₋₅ hydrocarbyl group.

5. The derivative according to claim 1, wherein R₂ represents a hydrogen atom, C₁₋₃ alkyl, or C₁₋₃ alkoxyl; R₃ represents a hydrogen atom, C₁₋₃ alkyl, or C₁₋₃ alkoxyl; R₄ represents a hydrogen atom, C₁₋₃ alkyl, or C₁₋₃ alkoxyl.

6. The derivative according to claim 1, wherein R₂ represents a methyl group; R₃ represents a hydrogen atom; R₄ represents a hydrogen atom, C₁₋₃ alkyl, or C₁₋₃ alkoxyl; R₅ represents a hydrogen atom

7. A pesticidal composition comprising 0.0001wt%-99.9wt% of a derivative (I) according to claim 1 or an agriculturally acceptable salt thereof in mixture with an agriculturally acceptable carrier or diluent.

8. A method of controlling pests which comprises applying to plants seeds, plant leaves, and/or plants fruits or the places where the plant is growing or is expected to be grown an insecticidally effective amount of a derivative of formula (I) according to claim 1.

9. The use of the derivative of formula (I) according to claims 1 in the preparation of a chemical insecticide for agriculture

10. A process for preparing the derivative of formula (I) according to claims 1, wherein the said process includes the following procedures:
(a) In an appropriate solvent, a compound of the formula (II) reacts with a compound of formula (III) at 60-100 °C to form a compound of formula (IV); 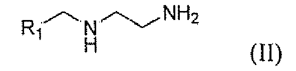 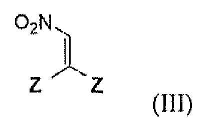 wherein Z represents Cl, -OR', -SR' in which R' represents C₁₋₃ alkyl, group (more preferably R'=methyl) 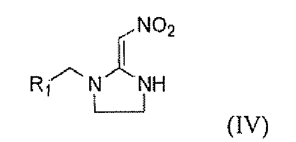
(b) In an appropriate solvent, the compound of formula (IV) reacts with a compound of formula (V) in the presence of an acid catalyst at 0-90 °C to form a compound of formula (VI) 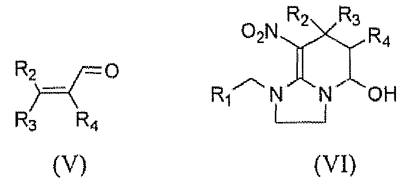
(c) In an appropriate solvent, the compound of the formula (VI) reacts with a compound of formula (VII) in the presence or a catalytic amount of acid at 30-70 °C to form a compound of formula (I),
R₅OH (VII)
wherein, R₁, R₂, R₃, R₄ and R₅ are defined as in claim 1;
provided that when Z represents -OR' or -SR¹, any two or three steps in procedures (a), (b) and (c) can be combined into one step
